# EUROPEAN PATENT APPLICATION

(11) **EP 4 269 572 A1**
(43) Date of publication of application: **01.11.2023**
(21) Application number: 21911106.9
(22) Date of filing: 24.12.2021
(51) Int. Cl.: C12N 9/04, C12N 15/10, C12N 15/53, C12P 7/42, C12P 13/00, C12P 17/12

(54) **MUTANT L-PIPECOLIC ACID HYDROXYLASE AND CIS-5-HYDROXY-L-PIPECOLIC ACID PRODUCTION METHOD UTILIZING SAME**

(30) Priority: 25.12.2020 JP 2020216765; 17.03.2021 JP 2021043441
(71) Applicant: API Corporation, Chikujo-gun, Fukuoka 871-0801 (JP)
(72) Inventor: ASANO, Yasuhisa, Imizu-shi, Toyama 939-0398 (JP); SHINODA, Suguru, Imizu-shi, Toyama 939-0398 (JP); ENOKI, Junichi, Imizu-shi, Toyama 939-0398 (JP); MIYAKE, Ryoma, Tokyo 100-8251 (JP); SASANO, Haruka, Tokyo 100-8251 (JP)
(74) Representative: Godemeyer Blum Lenze Patentanwälte Partnerschaft mbB - werkpatent
(86) International application number: PCT/JP2021/048406
(87) International publication number: WO 2022/138969

(57) **Abstract**

The present invention aims to provide a mutant L-pipecolic acid hydroxylase for highly productive and highly selective production of cis-5-hydroxy-L-pipecolic acid by hydroxylation of L-pipecolic acid, and to provide a novel method for industrially producing cis-5-hydroxy-L-pipecolic acid from L-pipecolic acid with high productivity at low cost. The present invention provides a mutant L-pipecolic acid hydroxylase having an amino acid sequence that a particular amino acid mutation(s) is/are introduced in the amino acid sequence of SEQ ID NO:2.

## Description

### TECHNICAL FIELD

The present invention relates to a mutant L-pipecolic acid hydroxylase, and a method of producing *cis*-5-hydroxy-L-pipecolic acid utilizing it. More specifically, the present invention relates to a mutant L-pipecolic acid hydroxylase having a high capacity to produce *cis*-5-hydroxy-L-pipecolic acid by hydroxylation of L-pipecolic acid, and a method of producing, with high productivity, *cis*-5-hydroxy-L-pipecolic acid from L-pipecolic acid utilizing this enzyme.

### BACKGROUND ART

*cis*-5-Hydroxy-L-pipecolic acid (which may be hereinafter referred to as "5HPA") is a compound useful as an intermediate for pharmaceuticals, and the like. It is known that *cis*-5-Hydroxy-L-pipecolic acid can be produced from L-pipecolic acid by a biological method.

For example, a protein (which may be hereinafter referred to as "SruPH") encoded by a polynucleotide (*cis* gene) expressed from 48 bases (corresponding to 16 amino acids) upstream of the annotation of CAC47686 protein derived from an alfalfa root nodule bacterium *Sinorhizobium meliloti* 1021 (which may be hereinafter referred to as "SmPH") or EFV12517 protein derived from *Segniliparus rugosus* ATCC BAA-974 is known to have *cis*-5-position hydroxylase activity on L-pipecolic acid, and to be capable of converting L-pipecolic acid to 5HPA.

However, these proteins were found not only to produce 5HPA, but also to produce, at several %, *cis*-3-hydroxypipecolic acid (which may be hereinafter referred to as "3HPA").

Under such circumstances, a protein having a high capacity to add a hydroxy group to the carbon atom at the 5-position (having a high position selectivity to the 5-position) of L-pipecolic acid has been demanded. As an L-pipecolic acid hydroxylase having a higher position selectivity to the 5-position than SmPH and SruPH, a protein derived from the *Xenorhabdus doucetiae* FRM16 strain (which may be hereinafter referred to as "XdPH") has been reported (Patent Document 1). However, XdPH has a problem in that, when it is expressed using *E. coli* as a host, it is expressed mostly as an inactive, insoluble protein.

From the viewpoint of the cost in industrial production, it is preferred that the enzyme protein can be expressed as an active enzyme or a soluble protein by a heterologous host such as *E. coli.* However, when an enzyme protein is expressed by a heterologous expression system, an enzyme which is naturally an active enzyme may be expressed as an inactive enzyme, or a protein which is naturally a soluble protein may be expressed as an insoluble protein.

An L-pipecolic acid hydroxylase desired in industrial production of 5HPA from L-pipecolic acid is an L-pipecolic acid hydroxylase which has L-pipecolic acid hydroxylation activity (ability to add a hydroxy group to the carbon atom at the 5-position of L-pipecolic acid), and which is expressed as a soluble protein, when it is expressed using *E. coli* as a host.

In view of this, a method of expressing an enzyme as an active mutant enzyme or soluble protein even in cases where the enzyme had not been expressed as an active enzyme or soluble protein, or had been expressed as an active enzyme only in a small amount in a heterologous expression system, has been reported (Patent Document 2). Patent Document 2 describes expression of an active mutant enzyme or a soluble protein by expressing, in a heterologous expression system, a gene having a base sequence encoding an amino acid sequence in which at least one hydrophobic amino acid present in a hydrophilic region of an α-helix structure portion is substituted (such that the target amino acid of substitution is substituted by an amino acid having higher hydrophilicity or lower hydrophobicity than the target amino acid), and/or in which at least one hydrophilic amino acid present in a hydrophobic region of an α-helix structure portion is substituted (such that the target amino acid of substitution is substituted by an amino acid having higher hydrophobicity or lower hydrophilicity).

### PRIOR ART DOCUMENTS

### [Patent Documents]

[Patent Document 1] WO 2016/076159
[Patent Document 2] WO 2016/199898

### SUMMARY OF THE INVENTION

### PROBLEMS TO BE SOLVED BY THE INVENTION

An object of the present invention to be achieved is to provide a mutant L-pipecolic acid hydroxylase for highly productive and highly selective production of *cis*-5-hydroxy-L-pipecolic acid by hydroxylation of L-pipecolic acid. Another object of the present invention to be achieved is to provide a novel method for industrially producing *cis*-5-hydroxy-L-pipecolic acid from L-pipecolic acid with high productivity at low cost.

### MEANS FOR SOLVING THE PROBLEMS

In order to achieve the objects, the present inventors intensively studied XdPH mutants with reference to the method described in Patent Document 2, and discovered that mutants having the amino acid sequence that the glutamic acid at position 15, the isoleucine at position 28, the valine at position 31, the cysteine at position 76, the isoleucine at position 108, the leucine at position 142, the glutamine at position 202, the alanine at position 239, the phenylalanine at position 246, and/or the isoleucine at position 271 is/are substituted by another/other amino acid(s) in the amino acid sequence of XdPH have a high capacity to selectively hydroxylate the carbon atom at the 5-position of L-pipecolic acid to produce *cis*-5-hydroxy-L-pipecolic acid (L-pipecolic acid hydroxylation activity), and that these mutants, when expressed using *E. coli* as a host, have high L-pipecolic acid hydroxylation activity and are expressed as soluble proteins, thereby reaching the present invention.

The present invention can be summarized as follows.
[1] A mutant L-pipecolic acid hydroxylase having an amino acid sequence that at least one amino acid mutation selected from the group consisting of the following (a) to (o) is introduced in the amino acid sequence of SEQ ID NO:2:
   (a) amino acid mutation in which the glutamic acid at position 15 is substituted by alanine;
   (b) amino acid mutation in which the isoleucine at position 28 is substituted by proline;
   (c) amino acid mutation in which the isoleucine at position 28 is substituted by arginine;
   (d) amino acid mutation in which the valine at position 31 is substituted by glutamic acid;
   (e) amino acid mutation in which the cysteine at position 76 is substituted by tyrosine;
   (f) amino acid mutation in which the isoleucine at position 108 is substituted by arginine;
   (g) amino acid mutation in which the leucine at position 142 is substituted by arginine;
   (h) amino acid mutation in which the leucine at position 142 is substituted by lysine;
   (i) amino acid mutation in which the leucine at position 142 is substituted by asparagine;
   (j) amino acid mutation in which the leucine at position 142 is substituted by glutamine;
   (k) amino acid mutation in which the leucine at position 142 is substituted by histidine;
   (l) amino acid mutation in which the glutamine at position 202 is substituted by proline;
   (m) amino acid mutation in which the alanine at position 239 is substituted by aspartic acid;
   (n) amino acid mutation in which the phenylalanine at position 246 is substituted by tyrosine; and
   (o) amino acid mutation in which the isoleucine at position 271 is substituted by glycine.
[2] The mutant L-pipecolic acid hydroxylase according to [1], having an amino acid sequence that at least two amino acid mutations selected from the group consisting of the (a) to (o) are introduced in the amino acid sequence of SEQ ID NO:2.
[3] The mutant L-pipecolic acid hydroxylase according to [1] or [2], having an amino acid sequence that at least three amino acid mutations selected from the group consisting of the (a) to (o) are introduced in the amino acid sequence of SEQ ID NO:2.
[4] A method of producing *cis*-5-hydroxy-L-pipecolic acid, the method comprising bringing the mutant L-pipecolic acid hydroxylase according to any one of [1] to [3], a microorganism or cell having a capacity to produce the enzyme, a processed product of the microorganism or cell, and/or a culture liquid containing the enzyme obtained by culturing the microorganism or cell, into contact with L-pipecolic acid, to produce *cis*-5-hydroxy-L-pipecolic acid.
[5] The method of producing *cis*-5-hydroxy-L-pipecolic acid according to [4], comprising bringing the mutant L-pipecolic acid hydroxylase, a microorganism or cell having a capacity to produce the enzyme, a processed product of the microorganism or cell, and/or a culture liquid containing the enzyme obtained by culturing the microorganism or cell, into contact with L-pipecolic acid in the presence of 2-oxoglutaric acid and ferrous ions.

### ADVANTAGEOUS EFFECTS OF THE INVENTION

The mutant L-pipecolic acid hydroxylase of the present invention has a high capacity to selectively hydroxylate the carbon atom at the 5-position of L-pipecolic acid to produce *cis*-5-hydroxy-L-pipecolic acid (L-pipecolic acid hydroxylation activity). Further, even in cases of expression using *E. coli* as a host, it has high L-pipecolic acid hydroxylation activity, and is expressed as a soluble protein. Thus, the mutant L-pipecolic acid hydroxylase of the present invention is useful for industrial production of *cis*-5-hydroxy-L-pipecolic acid from L-pipecolic acid.

Further, according to the method of producing *cis*-5-hydroxy-L-pipecolic acid of the present invention utilizing the mutant L-pipecolic acid hydroxylase, *cis*-5-hydroxy-L-pipecolic acid can be industrially produced from L-pipecolic acid with high productivity at low cost.

### BRIEF DESCRIPTION OF THE DRAWINGS

Fig. 1 is a diagram (electrophoretic profile) showing the result of evaluation of the solubility after low-temperature culture of XdPH mutants (introduction of a single mutation) in Example 6. In Fig. 1, the portion surrounded by a rectangle (dotted line) indicates XdPH mutants expressed in a soluble fraction.
Fig. 2 is a diagram (electrophoretic profile) showing the result of evaluation of the solubility after low-temperature culture or normal culture of XdPH mutants (introduction of a single mutation or multiple mutations) in Example 6. In Fig. 2, the portion surrounded by a rectangle (dotted line) indicates XdPH mutants expressed in a soluble fraction.
Fig. 3 is a diagram (electrophoretic profile) showing the result of evaluation of the solubility after low-temperature (15°C) culture of XdPH mutants in Example 7. In Fig. 3, the portion surrounded by a rectangle (dotted line) indicates XdPH mutants expressed in a soluble fraction.
Fig. 4 is a diagram (electrophoretic profile) showing the result of evaluation of the solubility after low-temperature (20°C) culture of XdPH mutants in Example 7. In Fig. 4, the portion surrounded by a rectangle (dotted line) indicates XdPH mutants expressed in a soluble fraction.
Fig. 5 is a diagram showing the result of HPLC analysis in Example 4.

### EMBODIMENTS FOR CARRYING OUT THE INVENTION

The present invention is described below in detail.

In the present description, "L-pipecolic acid hydroxylation activity" means an ability to add a hydroxy group to the carbon atom at the 5-position of L-pipecolic acid (an ability to convert L-pipecolic acid to *cis*-5-hydroxy-L-pipecolic acid).

In the present description, "L-pipecolic acid hydroxylation activity" can be evaluated by, for example, bringing the enzyme to be measured into contact with L-pipecolic acid, and measuring the amount of the *cis*-5-hydroxy-L-pipecolic acid generated by conversion from the L-pipecolic acid. More specifically, for example, a reaction liquid containing: L-pipecolic acid; 2-oxoglutaric acid, L-ascorbic acid, and ferrous ions as reaction aids; and the enzyme to be measured (or a microorganism or cell having a capacity to produce the enzyme, a processed product of the microorganism or cell, a culture liquid containing the enzyme obtained by culturing the microorganism or cell, or a protein purified from the microorganism or cell); is placed at an appropriate temperature (such as about 10°C to 45°C) and appropriate pressure (such as about atmospheric pressure), to allow the reaction to proceed, and then the amount of *cis*-5-hydroxy-L-pipecolic acid produced is measured. By this, the activity can be evaluated as the amount of *cis*-5-hydroxy-L-pipecolic acid produced (U/g, for example) per amount of cells placed in the reaction liquid (unit: g or turbidity) or total protein mass (unit: g). The unit (U) herein represents the capacity to produce 1 µmol of *cis*-5-hydroxy-L-pipecolic acid per minute.

In the present description, "enzyme" includes a purified enzyme (including a partially purified enzyme), and an enzyme immobilized on a support using a known immobilization technique, for example, an enzyme immobilized on a support such as polyacrylamide or carrageenan gel.

In the present description, whether or not the protein after heterologous expression is expressed as a soluble protein can be judged based on the amount of soluble protein in an extract after the heterologous expression. The measurement of the amount of the soluble protein can be carried out by, for example, chemically, physically, or mechanically destroying the microbial cells used for the heterologous expression (by treatment with a surfactant, sonication, grinding, french pressing, or the like), in the presence of a buffer, to obtain an extract, and then subjecting the extract to centrifugation, filtration, or the like to remove insoluble components such as insoluble protein contained in the extract, followed by measuring the amount of protein contained in the resulting supernatant. The measurement can be carried out by a known protein quantification method such as electrophoresis, the ELISA method, or Western blotting.

### 1. Mutant L-Pipecolic Acid Hydroxylase of Present Invention

The mutant L-pipecolic acid hydroxylase of the present invention has an amino acid sequence that at least one amino acid mutation selected from the group consisting of the following (a) to (o) is introduced in the amino acid sequence of SEQ ID NO:2:
(a) amino acid mutation in which the glutamic acid at position 15 is substituted by alanine;
(b) amino acid mutation in which the isoleucine at position 28 is substituted by proline;
(c) amino acid mutation in which the isoleucine at position 28 is substituted by arginine;
(d) amino acid mutation in which the valine at position 31 is substituted by glutamic acid;
(e) amino acid mutation in which the cysteine at position 76 is substituted by tyrosine;
(f) amino acid mutation in which the isoleucine at position 108 is substituted by arginine;
(g) amino acid mutation in which the leucine at position 142 is substituted by arginine;
(h) amino acid mutation in which the leucine at position 142 is substituted by lysine;
(i) amino acid mutation in which the leucine at position 142 is substituted by asparagine;
(j) amino acid mutation in which the leucine at position 142 is substituted by glutamine;
(k) amino acid mutation in which the leucine at position 142 is substituted by histidine;
(l) amino acid mutation in which the glutamine at position 202 is substituted by proline;
(m) amino acid mutation in which the alanine at position 239 is substituted by aspartic acid;
(n) amino acid mutation in which the phenylalanine at position 246 is substituted by tyrosine; and
(o) amino acid mutation in which the isoleucine at position 271 is substituted by glycine.

In the present invention, the amino acid sequence of SEQ ID NO:2 is an amino acid sequence of a protein (XdPH) derived from the *Xenorhabdus doucetiae* FRM16 strain, which protein is a wild-type L-pipecolic acid hydroxylase.

The mutant L-pipecolic acid hydroxylase of the present invention has an L-pipecolic acid hydroxylation activity equivalent to or higher than the activity of XdPH. Further, even in cases of expression using *E. coli* as a host, it has an L-pipecolic acid hydroxylation activity equivalent to or higher than the activity of XdPH, and is expressed as a soluble protein.

An amino acid sequence that at least one amino acid mutation selected from the group consisting of the (a) to (n) is introduced in the amino acid sequence of SEQ ID NO:2 tends to have high L-pipecolic acid hydroxylation activity and high solubility, which is preferred. In particular, an amino acid sequence in which at least one amino acid mutation selected from the group consisting of the (g) to (k) is introduced, that is, an amino acid sequence that the leucine at position 142 is substituted by another amino acid in the amino acid sequence of SEQ ID NO:2, is more preferred since it is thought to exhibit high L-pipecolic acid hydroxylation activity even in culture at a temperature around 28°C to 30°C, at which *E. coli* grows well. Examples of the other amino acid include an amino acid having a lower hydrophobicity than leucine, and having no negative charge. The amino acid mutation of (g) is more preferred.

An amino acid sequence that at least two amino acid mutations selected from the group consisting of the (a) to (o) are introduced in the amino acid sequence of SEQ ID NO:2 tends to have high L-pipecolic acid hydroxylation activity and high solubility, which is preferred.

The combination of the mutations is not limited, and examples of the combination include (g) and at least one except for (g), such as (g) and (a); (g) and (b); (g) and (c); (g) and (d); (g) and (e); and (g) and (n). Examples of the combination also include (b) and at least one except for (b), such as (b) and (e). Examples of the combination also include (e) and at least one except for (e), such as (e) and (b).

An amino acid sequence that at least three amino acid mutations selected from the group consisting of the (a) to (o) are introduced in the amino acid sequence of SEQ ID NO:2 has even higher L-pipecolic acid hydroxylation activity, which is more preferred.

The combination of the mutations is not limited, and examples of the combination include (g) and any two except for (g), such as (g) and two selected from (a), (b), (c), (d), (e), and (n), for example, the combination of (g), (b), and (e); (g), (b), and (d); or (g), (b), and (n).

The mutant L-pipecolic acid hydroxylase of the present invention can be produced from the amino acid sequence of SEQ ID NO:2 by a method known to those skilled in the art, for example, a well-known technique such as site-directed mutagenesis or PCR.

The mutant L-pipecolic acid hydroxylase of the present invention can also be produced by culturing a transformant containing a nucleic acid encoding it, and separating and purifying the mutant L-pipecolic acid hydroxylase from the obtained culture. The nucleic acid encoding the mutant L-pipecolic acid hydroxylase of the present invention may be either DNA or RNA, or a DNA/RNA chimera. The nucleic acid is preferably DNA. The nucleic acid may be either a single-stranded or double-stranded nucleic acid. In cases where the nucleic acid is a double-stranded nucleic acid, it may be a double-stranded DNA, double-stranded RNA, or DNA: RNA hybrid. In cases where the nucleic acid is a single-stranded nucleic acid, it may be either a sense strand (that is, a coding strand) or an antisense strand (that is, a non-coding strand).

Examples of the original DNA from which the DNA encoding the mutant L-pipecolic acid hydroxylase of the present invention is derived include a DNA cloned from the *Xenorhabdus doucetiae* FRM16 strain. The original DNA can be obtained by, for example, known PCR or hybridization from a DNA fraction prepared from cells or a tissue derived from the *Xenorhabdus doucetiae* FRM16 strain. Examples of the original DNA also include a full-length L-pipecolic acid hydroxylase cDNA directly amplified by reverse transcriptase-PCR using, as a template, total RNA or an mRNA fraction prepared from cells or a tissue derived from the *Xenorhabdus doucetiae* FRM16 strain. By converting (introducing a mutation(s) to) such a DNA using a known kit, for example, Mutan-super Express Km (TAKARABIO INC.) or Mutan-K (TAKARA BIO INC.) according to a method known per se such as the ODA-LA PCR method, Gapped duplex method, or Kunkel method, or according to a method equivalent thereto, the DNA encoding the mutant L-pipecolic acid hydroxylase of the present invention can be obtained. The DNA can also be obtained by preparing a cDNA library by inserting the total RNA or mRNA fragments described above into an appropriate vector, cloning a cDNA from the cDNA library by the colony or plaque hybridization method, PCR method, or the like, and then converting the cloned cDNA according to the method described above. The vector used for the library may be any of bacteriophages, plasmids, cosmids, phagemids, and the like.

Examples of the nucleic acid encoding the protein having the amino acid sequence of SEQ ID NO:2 include a nucleic acid containing the base sequence of SEQ ID NO: 1. The base sequence of SEQ ID NO: 1 is a synthetic base sequence obtained by subjecting the gene encoding the amino acid sequence of SEQ ID NO:2 in the *Xenorhabdus doucetiae* FRM16 strain to codon optimization for expression in *E. coli.* Examples of the nucleic acid encoding the protein having L-pipecolic acid hydroxylation activity in the present invention include not only the gene in *Xenorhabdus doucetiae,* but also, of course, nucleic acids obtained by such codon optimization in accordance with the host to be transformed.

Those skilled in the art can introduce a base substitution(s) that cause(s) a desired mutation(s) by appropriately carrying out substitution, deletion, insertion, and/or addition for the nucleic acid of SEQ ID NO: 1 using site-directed mutagenesis (Nucleic Acids Res. 10, pp. 6487 (1982); Methods in Enzymol. 100, pp. 448 (1983); Molecular Cloning, PCR A Practical Approach IRL Press pp. 200 (1991)) or the like, to obtain a nucleic acid encoding the amino acid sequence of a mutant L-pipecolic acid hydroxylase containing a mutation(s) of (a) to (o).

In the later-described production method of the present invention, the mutant L-pipecolic acid hydroxylase may be directly used for the reaction with the substrate L-pipecolic acid, but it is preferred to use a microorganism or cell having a capacity to produce the enzyme, a processed product of the microorganism or cell, and/or a culture liquid containing the enzyme obtained by culturing the microorganism or cell.

The microorganism or cell having a capacity to produce the mutant L-pipecolic acid hydroxylase of the present invention may be a microorganism or cell originally having a capacity to produce the mutant L-pipecolic acid hydroxylase, or may be a microorganism or cell prepared by imparting the production capacity by breeding. The microorganism or cell may be either a live or dead microorganism or cell. For example, a dormant microbial cell or the like may be preferably used. Examples of the type of the microorganism or cell having a capacity to produce the mutant L-pipecolic acid hydroxylase of the present invention include those described later as "host microorganism" or "host cell".

As the means for imparting the production capacity by breeding, a known method such as genetic recombination treatment (transformation) or mutagenesis may be employed. Examples of the method of the transformation include a method in which the desired DNA is introduced, and a method in which an expression regulation sequence such as a promoter on the chromosome is modified to enhance expression of the desired DNA.

Among these, a microorganism or cell transformed with a DNA encoding the protein (mutant L-pipecolic acid hydroxylase) of the present invention is preferably used.

The nucleic acid (DNA) encoding the mutant L-pipecolic acid hydroxylase of the present invention can be obtained as described above by, for example, carrying out cloning by PCR using chromosomal DNA derived from the *Xenorhabdus doucetiae* FRM16 strain as a template, and using appropriate primers, followed by converting the cloned product.

The nucleic acid (DNA) encoding the mutant L-pipecolic acid hydroxylase of the present invention can also be obtained as described above by, for example, directly amplifying a full-length mutant L-pipecolic acid hydroxylase cDNA by RT-PCR using, as a template, total RNA or mRNA derived from the *Xenorhabdus doucetiae* FRM16 strain, and then cloning the prepared cDNA by PCR using appropriate primers, followed by converting the cloned product.

For example, by inserting the thus obtained DNA encoding the mutant L-pipecolic acid hydroxylase of the present invention into a known expression vector in a manner which allows expression of the mutant L-pipecolic acid hydroxylase, a protein gene expression vector in the present invention is provided. By transforming a host cell with this expression vector, a transformant in which the DNA encoding the protein of the present invention is introduced can be obtained. The transformant can also be obtained by incorporating a DNA encoding the protein of the present invention into the chromosomal DNA of a host in a manner which allows expression of the protein, by a method such as homologous recombination.

The "expression vector" in the present description is a genetic factor used for replicating and expressing a protein having a desired function in a host organism. A polynucleotide encoding the protein having the desired function is incorporated in the vector, and the resulting vector is introduced into the host cell. Examples of the expression vector include, but are not limited to, plasmids, viruses, phages, and cosmids. The expression vector is preferably a plasmid.

The "transformant" in the present description means a microorganism or cell in which a desired gene is introduced using the expression vector or the like, and which, as a result, is capable of exhibiting a desired character related to a protein having a desired function.

Specific examples of the method for preparing the transformant include a method in which a DNA encoding the protein of the present invention (mutant L-pipecolic acid hydroxylase) is introduced into a plasmid vector, phage vector, or virus vector which can be stably present in a host cell, followed by introducing the constructed expression vector into the host cell, and a method in which the DNA is directly introduced into the host genome, followed by allowing transcription and translation of the genetic information. In this case, in the host, an appropriate promoter is preferably linked 5'-upstream of the DNA, and, in addition, a terminator is more preferably linked 3'-downstream of the DNA. Such a promoter and a terminator are not limited as long as they are a promoter and a terminator known to function in the cell used as the host. For example, a vector, a promoter, and a terminator described in detail in "Fundamental Microbiology 8: Genetic Engineering, Kyoritsu Shuppan Co., Ltd." may be used.

The host microorganism to be transformed for the expression of the mutant L-pipecolic acid hydroxylase of the present invention is not limited as long as the host itself does not adversely affect the substrate L-pipecolic acid or cis-5-hydroxy-L-pipecolic acid, which is the desired product. Specific examples of the host microorganism include the following microorganisms:
bacteria belonging to the genera *Escherichia, Bacillus, Pseudomonas, Serratia, Brevibacterium, Corynebacterium, Streptococcus, Lactobacillus,* and the like whose host vector systems have been established;
actinomycetes belonging to the genera *Rhodococcus, Streptomyces,* and the like whose host vector systems have been established;
yeasts belonging to the genera *Saccharomyces, Kluyveromyces, Schizosaccharomyces, Zygosaccharomyces, Yarrowia, Trichosporon, Rhodosporidium, Hansenula, Pichia, Candida,* and the like whose host vector systems have been established; and
molds belonging to the genera *Neurospora, Aspergillus, Cephalosporium, Trichoderma,* and the like whose host vector systems have been established.

The procedure for the preparation of the transformant, the method for construction of the recombinant vector suitable for the host, and the method for culturing the host can be carried out according to techniques commonly used in the fields of molecular biology, bioengineering, and genetic engineering (for example, methods described in Green et al., Molecular Cloning: A Laboratory Manual (4th ed.) Cold Spring Harbor Press, Cold Spring Harbor, NY (2012)).

The following are specific examples of preferred host microorganisms; and transformation methods, vectors, promoters, terminators, and the like preferred for such microorganisms. However, the present invention is not limited to these examples.

For the genus *Escherichia,* especially *Escherichia coli,* examples of the plasmid vector include pBR and pUC plasmids, and examples of the promoter include promoters derived from *lac* (β-galactosidase), *trp* (tryptophan operon), *tac, trc* (fusion of *lac* and *trp*), λ phage PL or PR, T7 phage, or the like. Examples of the terminator include terminators derived from *trpA,* phages, or *rrnB* ribosomal RNA.

For the genus *Bacillus,* examples of the vector include pUB 110 plasmids and pC194 plasmids. Integration into the chromosome is also possible. Examples of the promoter and the terminator that may be used include those of the genes of enzymes such as alkaline protease, neutral protease, and α-amylase.

For the genus *Pseudomonas,* examples of the vector include common host vector systems established in *Pseudomonasputida, Pseudomonas cepacia,* and the like; and the wide-host-range vector (containing genes required for autonomous replication derived from RSF1010 and the like) pKT240, which is based on a plasmid involved in degradation of toluene compounds, TOL plasmid (Gene, 26, 273-82 (1983)).

For the genus *Brevibacterium,* especially *Brevibacterium lactofermentum,* examples of the vector include plasmid vectors such as pAJ43 (Gene 39, 281 (1985)). Examples of the promoter and the terminator that may be utilized include promoters and terminators used in *E. coli.*

For the genus *Corynebacterium,* especially *Corynebacterium glutamicum,* examples of the vector include plasmid vectors such as pCS11 (JP 57-183799 A) and pCB101 (Mol. Gen. Genet. 196, 175 (1984)).

For the genus *Saccharomyces,* especially *Saccharomyces cerevisiae,* examples of the vector include YRp, YEp, YCp, and YIp plasmids. Examples of the promoter and the terminator that may be used include those of the genes of enzymes such as alcohol dehydrogenase, glyceraldehyde-3-phosphate dehydrogenase, acid phosphatase, β-galactosidase, phosphoglycerate kinase, and enolase.

For the genus *Schizosaccharomyces,* examples of the vector include the plasmid vectors derived from *Schizosaccharomyces pombe* described in Mol. Cell. Biol. 6, 80 (1986). Among these, pAUR224 is commercially available from Takara Bio Inc., and can be easily used.

In the genus *Aspergillus, Aspergillus niger, Aspergillus oryzae,* and the like are best studied among the molds. For these, integration into a plasmid or the chromosome can be utilized, and promoters derived from extracellular protease or amylase can be utilized (Trends in Biotechnology 7, 283-287 (1989)).

Host-vector systems other than the above-described systems have also been established for various microorganisms, and those systems may be used as appropriate.

Various host-vector systems have been established for plants and animals besides microorganisms. In particular, systems that allow expression of a large amount of foreign protein in an animal such as an insect (for example, a silkworm) (Nature 315, 592-594 (1985)), or in a plant such as rapeseed, maize, or potato; and systems using a cell-free protein synthesis system such as an *E. coli* cell-free extract or a wheat germ; have been established, and may be preferably used.

Examples of the processed product of the microorganism or cell having a capacity to produce the mutant L-pipecolic acid hydroxylase of the present invention include cell preparations, for example, preparations obtained by treatment of the microorganism or cell with an organic solvent such as acetone, dimethyl sulfoxide (DMSO), or toluene, or with a surfactant, preparations obtained by freeze-drying of the microorganism or cell, and preparations obtained by physical or enzymatic destruction of the microorganism or cell; enzyme fractions of the microorganism or cell obtained as crude products or purified products; and products prepared by immobilizing these on a support represented by polyacrylamide gel or carrageenan gel.

Examples of the culture liquid containing the enzyme obtained by culturing the microorganism or cell having a capacity to produce the mutant L-pipecolic acid hydroxylase of the present invention include suspensions containing the microorganism or cell and a liquid medium, and also include, when the microorganism or cell is a secretory-expression-type cell, supernatants obtained by removing the microorganism or cell by centrifugation or the like, and concentrates thereof. The culture conditions may be conditions suitable for culture of the microorganism or cell, or may be conditions appropriately adjusted to optimize the activity, physical properties, productivity, or the like of the mutant L-pipecolic acid hydroxylase of the present invention. For example, regarding the conditions for culture using *E. coli* as a host, the culture is carried out, for example, at a temperature of usually 15°C to 37°C for about 12 hours to 48 hours. In some cases, low-temperature culture is preferred from the viewpoint of the enzyme activity and the solubility. In such cases, the culture is preferably carried out at a temperature of 15°C to 25°C, or 15°C to 20°C.

### 2. Method of Producing cis-5-Hydroxy-L-Pipecolic Acid of Invention

The method of producing *cis*-5-hydroxy-L-pipecolic acid of the present invention comprises bringing the mutant L-pipecolic acid hydroxylase of the present invention, a microorganism or cell having a capacity to produce the enzyme, a processed product of the microorganism or cell, and/or a culture liquid containing the enzyme obtained by culturing the microorganism or cell (which may be hereinafter collectively referred to as "mutant L-pipecolic acid hydroxylase of the present invention or the like"), into contact with L-pipecolic acid to allow the reaction.

The production method of the present invention may use a purified or crudely purified product of the mutant L-pipecolic acid hydroxylase of the present invention, a microorganism or cell having a capacity to produce the mutant L-pipecolic acid hydroxylase of the present invention (such as a transformant having a DNA encoding the mutant L-pipecolic acid hydroxylase of the present invention), a processed product of the microorganism or cell, and/or a culture liquid containing the enzyme obtained by culturing the microorganism or cell. Among these, it is preferred to use the microorganism or cell having a capacity to produce the mutant L-pipecolic acid hydroxylase of the present invention (such as a transformant having a DNA encoding the mutant L-pipecolic acid hydroxylase of the present invention), the processed product of the microorganism or cell, and/or the culture liquid containing the enzyme obtained by culturing the microorganism or cell. It is more preferred to use the transformant having a DNA encoding the mutant L-pipecolic acid hydroxylase of the present invention.

In the production method of the present invention, multiple kinds of mutant L-pipecolic acid hydroxylases may be used in combination.

The amount of the mutant L-pipecolic acid hydroxylase of the present invention or the like to be brought into contact with L-pipecolic acid is not limited as long as cis-5-hydroxy-L-pipecolic acid can be produced therewith. For example, in cases where the microorganism or cell is added to a reaction liquid containing L-pipecolic acid, the microorganism or cell is added such that its concentration in the reaction liquid becomes usually about 0.1 w/v% to 50 w/v%, preferably 1 w/v% to 20 w/v% in terms of the wet microbial cell weight. In cases where the processed product or culture liquid is added to a reaction liquid containing L-pipecolic acid, the specific activity of the mutant L-pipecolic acid hydroxylase used is determined, and the processed product or culture liquid is added in an amount with which the concentration of the microorganism or cell in the reaction liquid becomes the above-described concentration. Here, w/v% represents weight/volume%.

The contacting method is not limited. For example, the substrate L-pipecolic acid may be added to a liquid containing the mutant L-pipecolic acid hydroxylase of the present invention or the like. Alternatively, the mutant L-pipecolic acid hydroxylase of the present invention or the like may be added to a liquid containing the substrate (reaction substrate) L-pipecolic acid. By the contact between the L-pipecolic acid and the mutant L-pipecolic acid hydroxylase of the present invention or the like, hydroxylation of the L-pipecolic acid occurs to produce *cis*-5-hydroxy-L-pipecolic acid.

The amount of the substrate L-pipecolic acid may be appropriately selected depending on the amount of the *cis*-5-hydroxy-L-pipecolic acid to be produced. The L-pipecolic acid may be used within the range of usually 0.01 w/v% to 90 w/v%, preferably 0.1 w/v% to 30 w/v% in terms of the substrate concentration in a liquid containing the L-pipecolic acid and the mutant L-pipecolic acid hydroxylase of the present invention or the like (which may be hereinafter referred to as "reaction liquid").

The L-pipecolic acid and the mutant L-pipecolic acid hydroxylase of the present invention or the like may be added at once at the beginning of the reaction, or may be added continuously or intermittently from the viewpoint of reducing the effect of substrate inhibition of the enzyme, if any, and increasing the concentration of the accumulated product.

The contacting is preferably carried out in the presence of 2-oxoglutaric acid and ferrous ions.

The 2-oxoglutaric acid is added in an amount of usually one or more moles, preferably within the range of one to two moles, per mole of the substrate L-pipecolic acid. The 2-oxoglutaric acid may be added at once at the beginning of the reaction, or may be added continuously or intermittently from the viewpoint of reducing an inhibitory action on the enzyme, if any, and increasing the concentration of the accumulated product. Alternatively, an inexpensive compound metabolizable by the host, such as glucose, may be added instead of the 2-oxoglutaric acid, to allow the host to metabolize it, and 2-oxoglutaric acid produced during this process may be used for the reaction.

The ferrous ions may be used in an amount within the range of usually 0.001 mmol/L to 100 mmol/L, preferably 0.01 mmol/L to 50 mmol/L, especially preferably 0.1 mmol/L to 10 mmol/L in terms of the concentration in the reaction liquid. The ferrous ions may be added at once as iron sulfate or the like at the beginning of the reaction. Further supplementation of ferrous ions is effective in cases where the added ferrous ions are oxidized into ferric ions or decreased due to formation of a precipitate during the reaction. In cases where the mutant L-pipecolic acid hydroxylase of the present invention or the like already contains a sufficient amount of ferrous ions, addition of ferrous ions is not necessarily required.

The contacting is preferably carried out in the presence of also L-ascorbic acid. The L-ascorbic acid may be used in an amount within the range of usually 0.001 mmol/L to 50 mmol/L, preferably 0.01 mmol/L to 30 mmol/L, especially preferably 0.1 mmol/L to 25 mmol/L in terms of the concentration in the reaction liquid. By the addition of L-ascorbic acid, oxidation of the ferrous ions can be reduced.

The contacting may be carried out usually in an aqueous medium, or a mixture of an aqueous medium and an organic solvent. From the viewpoint of the post-reaction processing, and from the industrial point of view, the contacting is preferably carried out in an aqueous medium.

Examples of the aqueous medium include water; and known buffers such as Good's buffer, phosphate buffer, Tris buffer, and borate buffer. Examples of the organic solvent that may be used include those in which the substrate L-pipecolic acid is highly soluble, such as methanol, ethanol, 1-propanol, 2-propanol, 1-butanol, *tert*-butanol, acetone, and dimethyl sulfoxide. Other examples of the organic solvent that may be used include those effective for, for example, removal of a reaction by-product. Examples of such an organic solvent that may be used include ethyl acetate, butyl acetate, toluene, chloroform, and *n*-hexane.

The contacting may be carried out under near-atmospheric pressure within the temperature range of usually 4°C to 60°C, preferably 10°C to 45°C, especially preferably 15°C to 40°C. The contacting may be carried out under conditions usually at a pH of 3 to 11, preferably at a pH of 5 to 8. The contacting time is not limited as long as the L-pipecolic acid is hydroxylated to produce *cis*-5-hydroxy-L-pipecolic acid. The contacting time is usually not less than 10 minutes, preferably not less than 30 minutes, and is usually not more than 90 hours, preferably not more than 72 hours.

The produced *cis*-5-hydroxy-L-pipecolic acid may be subjected to separation of microbial cells, proteins, and the like in the reaction liquid by a separation or purification method known to those skilled in the art, such as centrifugation or membrane treatment, and then to purification by an appropriate combination of methods known to those skilled in the art, for example, extraction using an organic solvent such as 1-butanol or *tert*-butanol, distillation, column chromatography using an ion-exchange resin, silica gel, or the like, crystallization at the isoelectric point, and/or crystallization with monohydrochloride, dihydrochloride, calcium salt, or the like.

By the production method of the present invention, *cis*-5-hydroxy-L-pipecolic acid, which is useful as, for example, an intermediate for pharmaceuticals, can be industrially produced with high productivity at low cost.

### EXAMPLES

The present invention is described below in more detail by way of Examples, but the present invention is not limited to Examples.

In Examples, amino acids may be abbreviated as follows.

**Table 1**

| Amino acid | Abbreviation |
|---|---|
| Alanine | A |
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

### <Example 1: Obtaining of XdPH Gene and Introduction of Mutations to XdPH Gene 1>

A gene sequence encoding L-proline *cis*-4-hydroxylase XdPH (Gen Bank Accession No. CDG16639; SEQ ID NO:2) derived from the *Xenorhabdus doucetiae* FRM16 strain, codon-optimized for expression in *E. coli* (xdph_Ecodon; SEQ ID NO: 1), was artificially synthesized by DNA 2.0 Inc. (currently ATUM), and then inserted into pJExpress411 (manufactured by DNA 2.0 Inc.), to prepare the plasmid pJ411XdPH. The obtained plasmid pJ411XdPH was used as a template to prepare mutant expression plasmids shown in Table 1 (Nos. m1 to m25, and m37 to m59) according to a conventional method.

### <Example 2: Introduction of Mutations to XdPH Gene 2>

The plasmid pJ411XdPH_m15, obtained in Example 1 and having a mutant gene in which the leucine at position 142 is substituted by arginine, was used as a template to obtain the plasmid pJ411XdPH_m26, which expresses a double mutant, by introduction of an additional mutation by the same method as in Example 1. The plasmid has a gene encoding the mutant enzyme XdPHm26, in which the leucine at position 142 is substituted by arginine, and in which the glutamic acid at amino acid position 15 is substituted by alanine. Similarly, mutant expression plasmids shown in Table 2 (pJ411XdPH_m27 to pJ411XdPH_m36) were prepared.

**Table 2: Prepared mutant expression plasmids**

| Mutant No. | Gene mutation site | Template plasmid | Mutant expression plasmid |
|---|---|---|---|
| m1 | L89R | pJ411XdPH | pJ411XdPH m1 |
| m2 | L208E | pT41 IXdPH | pJ411XdPH m2 |
| m3 | H218V | pJ411XdPH | pT411XdPH m3 |
| m4 | D2501 | pT411XdPH | pJ411XdPH m4 |
| m5 | R262A | pJ411XdPH | pJ411XdPH m5 |
| m6 | R262V | pI41 IXdPH | pJ411XdPH m6 |
| m7 | 1271G | p1411XdPH | pJ411XdPH m7 |
| m8 | I271Y | pJ411XdPH | pJ411XdPH m8 |
| m9 | E15A | pJ411XdPH | pJ411XdPH m9 |
| m10 | 128P | pJ411XdPH | pJ411XdPH m10 |
| m11 | 128R | pJ411XdPH | pJ411XdPH m11 |
| m12 | V31L | pJ411XdPH | pJ411XdPH m12 |
| m13 | C76Y | pJ411XdPH | pJ411XdPH m13 |
| m14 | 1108R | pJ411XdPH | pJ411XdPH m14 |
| m15 | L142R | pJ411XdPH | pJ411XdPH m15 |
| m16 | C189D | pT411XdPH | pJ411XdPH m16 |
| m17 | R209L | p1411XdPH | p1411XdPH m17 |
| m18 | R243G | pT411XdPH | pT411XdPH m18 |
| m19 | E244A | pJ411XdPH | pJ411XdPH m19 |
| m20 | Y245A | p1411XdPH | pJ411XdPH m20 |
| m21 | Y245V | pJ411XdPH | pJ411XdPH m21 |
| m22 | F246W | pJ411XdPH | pJ411XdPH m22 |
| m23 | F246Y | pJ411XdPH | pJ411XdPH m23 |
| m24 | L248W | pJ411XdPH | pJ411XdPH m24 |
| m25 | C62D | pJ411XdPH | pJ411XdPH m25 |
| m26 | E15A + L142R | pJ411XdPH m15 | pJ411XdPH m26 |
| m27 | 128P + L142R | pJ411XdPH m15 | pJ411XdPH m27 |
| m28 | 128R + L142R | pJ411XdPH m15 | pJ411XdPH m28 |
| m29 | C76Y + L142R | pT411XdPH m15 | pJ411XdPH m29 |
| m30 | V31E + L142R | pJ411XdPH m15 | pJ411XdPH m30 |
| m31 | F246Y + L142R | pJ411XdPH m15 | pJ411XdPH m31 |
| m32 | V62D + L142R | pJ411XdPH m15 | pJ411XdPH m32 |
| m33 | 128P + C76Y | pJ411XdPH m10 | pJ411XdPH m33 |
| m34 | 128P + C76Y + L142R | pJ411XdPH m27 | pJ411XdPH m34 |
| m35 | 128P + V3 1E + L142R | pJ411XdPH m27 | pJ411XdPH m35 |
| m36 | 128P + L142R + F246Y | pJ411XdPH m27 | pJ411XdPH m36 |
| m37 | A32E | pJ411XdPH | pJ411XdPH m37 |
| m38 | I44H | pJ411XdPH | pJ411XdPH m38 |
| m39 | S98L | pJ411XdPH | pJ411XdPH m39 |
| m40 | Y105I | pJ411XdPH | pJ411XdPH m40 |
| m41 | V123P | pJ411XdPH | pJ411XdPH m41 |
| m42 | V163P | pJ411XdPH | pJ411XdPH m42 |
| m43 | Y166L | pJ411XdPH | pJ411XdPH m43 |
| m44 | K177A | pJ411XdPH | pJ411XdPH m44 |
| m45 | V190G | pJ411XdPH | pJ411XdPH m45 |
| m46 | 1197R | pJ411XdPH | pT411XdPH m46 |
| m47 | H199V | pJ411XdPH | pJ411XdPH m47 |
| m48 | F222E | pJ411XdPH | p1411XdPH m48 |
| m49 | A229R | pT411XdPH | pJ411XdPH m49 |
| m50 | A239D | pJ411XdPH | pJ411XdPH m50 |
| m51 | 1232E | pJ411XdPH | pJ411XdPH m51 |
| m52 | D75P | pJ411XdPH | pJ411XdPH m52 |
| m53 | G151A | pJ411XdPH | pJ411XdPH m53 |
| m54 | A154P | pJ411XdPH | pJ411XdPH m54 |
| m55 | K165R | pJ411XdPH | pJ411XdPH m55 |
| m56 | O202P | pJ411XdPH | pJ411XdPH m56 |
| m57 | C2521 | pJ411XdPH | pJ411XdPH m57 |
| m58 | P257G | pJ411XdPH | pJ411XdPH m58 |
| m59 | Y258D | pJ411XdPH | pJ411XdPH m59 |

### <Example 3: Obtaining of Mutant XdPH Gene-Expressing Bacterial Cells 1>

Using each of the wild-type plasmid pJ411XdPH, and the mutant expression plasmids obtained in Examples 1 and 2, *E. coli (Escherichia coli*) BL21(DE3) (manufactured by Invitrogen) was transformed according to a conventional method, to obtain each recombinant *E. coli.*

### <Example 4: Evaluation of Activity of Mutant L-Pipecolic Acid Hydroxylases (XdPH Mutants) 1>

### (1) Providing of Enzyme Liquids

In order to obtain bacterial cells expressing the introduced gene, each recombinant *E. coli* obtained in Example 3 (the recombinant *E. coli* obtained by transformation using each of the wild-type plasmid pJ411XdPH, and the mutant expression plasmids pJ411XdPH_m1 to pJ411XdPH_m31, and pJ411XdPH_m33 to pJ411XdPH_m36) was precultured at 30°C for about 5 hours using 1 mL of liquid LB medium supplemented with kanamycin and a *lac* promoter inducer. Thereafter, normal culture or low-temperature culture was carried out, and the bacterial cells were collected. In cases of the normal culture, the preculture was carried out, and then culture was carried out at 28°C or 30°C for about 20 hours, followed by collection of the bacterial cells. In cases of the low-temperature culture, the preculture was carried out, and then culture was carried out at 15°C for about 24 hours, followed by collection of the bacterial cells.

Subsequently, 0.6 mL of each recombinant *E. coli* was centrifuged to collect the bacterial cells, and the collected bacterial cells were suspended in 50 mmol/L MES (2-morpholinoethanesulfonic acid) buffer at pH 6.5. A container containing 0.5 mL of the obtained suspension was placed in ice water, and sonication was carried out, followed by centrifugation at a rotation speed of 12,000 rpm. The obtained supernatant was used as an enzyme liquid for evaluation of the L-pipecolic acid hydroxylation activity.

### (2) Evaluation of Activity

In a plastic tube, 0.2 mL of a reaction liquid prepared by mixing components at the concentrations shown in Table 3 was placed. The tube was then shaken at 30°C for 30 minutes.

**Table 3**

| Component | Concentration |
|---|---|
| L-Pipecolic acid | 20 mmol/L |
| 2-Oxoglutaric acid | 40 mmol/L |
| L-Ascorbic acid | 1 mmol/L |
| Iron sulfate | 0.5 mmol/L |
| Each enzyme liquid provided in (1) above | About 2 mg/mL in terms of total protein concentration |

To the reaction liquid after the shaking, 0.1 mL of hydrochloric acid having a concentration of 1 mmol/L was added to stop the reaction. Thereafter, 0.1 mL of aqueous sodium hydroxide solution having a concentration of 1 mmol/L was added thereto to neutralize the reaction liquid. The reaction liquid after the neutralization was centrifuged, and the resulting precipitate was removed to obtain a solution. The solution was then analyzed by HPLC (High Performans Liquid Chromatography) under the following conditions, to measure the concentration of the produced *cis*-5-hydroxy-L-pipecolic acid.

### <HPLC Analysis Conditions>

Apparatus used: Chromaster (registered trademark) (manufactured by Hitachi High-Tech Science Corporation)
Column: Astec (registered trademark) CLC-D Chiral HPLC Column; 5 µm; 150 × 4.6 mm
Detection wavelength: 254 nm
Injection volume: 10 µL
Eluent: 2 mmol/L copper sulfate
Flow rate: 1 mL/min
Column temperature: 45°C

The evaluation results on the L-pipecolic acid hydroxylation activity of the wild-type enzyme XdPH and the XdPH mutants are shown in Table 4 [Low-temperature culture] and Table 5 [Normal culture].

In Table 4 and Table 5, the L-pipecolic acid hydroxylation activity was evaluated as the amount of *cis*-5-hydroxy-L-pipecolic acid produced (U/g-total protein) per total protein mass (unit: g). The unit (U) herein represents the capacity to produce 1 µmol of *cis*-5-hydroxy-L-pipecolic acid per minute. In Table 4 and Table 5, the symbols indicating the L-pipecolic acid hydroxylation activity have the following meanings.

| Symbol | Amount of *cis*-5-hydroxy-L-pipecolic acid produced (U/g-total protein) |
|---|---|
| - | 0 |
| + | 1 to 5 |
| ++ | 5 to 10 |
| +++ | 10 to 50 |
| ++++ | 50 to 100 |
| +++++ | 100 to 150 |

**Table 4: Low-temperature culture**

| Mutant No. | Mutation site | Culture Temp. | L-Pipecolic acid hydroxylation activity |
|---|---|---|---|
| Wild-type XdPH | (No mutation) | 15°C | + |
| m1 | L89R | 15°C | + |
| m2 | L208E | 15°C | + |
| m3 | H218V | 15°C | + |
| m4 | D2501 | 15°C | + |
| m5 | R262A | 15°C | + |
| m6 | R262V | 15°C | + |
| m7 | I271G | 15°C | + |
| m8 | I271Y | 15°C | + |
| m9 | E15A | 15°C | ++ |
| m10 | 128P | 15°C | ++ |
| m11 | 128R | 15°C | ++ |
| m12 | V31E | 15°C | ++ |
| m13 | C76Y | 15°C | ++ |
| m14 | 1108R | 15°C | ++ |
| m15 | L142R | 15°C | +++ |
| m16 | C189D | 15°C | + |
| m17 | R209L | 15°C | + |
| m18 | R243G | 15°C | + |
| m19 | E244A | 15°C | + |
| m20 | Y245A | 15°C | + |
| m21 | Y245V | 15°C | + |
| m22 | F246W | 15°C | + |
| m23 | F246Y | 15°C | ++ |
| m24 | L248W | 15°C | + |
| m25 | V62D | 15°C | + |
| m26 | E15A + L142R | 15°C | +++ |
| m27 | I28P + L142R | 15°C | +++ |
| m28 | 128R + L142R | 15°C | +++ |
| m29 | C76Y + L142R | 15°C | +++ |
| m30 | V31E + L142R | 15°C | +++ |
| m31 | F246Y + L142R | 15°C | +++ |
| m33 | 128P + C76Y | 15°C | +++ |
| m34 | 128P + C76Y + L142R | 15°C | ++++ |
| m35 | 128P + V31E + L142R | 15°C | +++ |
| m36 | I28P + L142R + F246Y | 15°C | +++ |

**Table 5: Normal culture**

| Mutant No. | Mutation site | Culture Temp. | L-Pipecolic acid hydroxylation activity |
|---|---|---|---|
| Wild-type XdPH | (No mutation) | 28°C | - |
| | | 30°C | - |
| m1 | L89R | 28°C | - |
| m12 | V31E | 28°C | - |
| m13 | C76Y | 28°C | - |
| m14 | I108R | 28°C | - |
| m15 | L142R | 28°C | ++ |
| m16 | C189D | 28°C | - |
| m21 | Y245V | 28°C | - |
| m23 | F246Y | 28°C | - |
| m26 | E15A + L142R | 28°C | +++ |
| m27 | I28P + L142R | 30°C | +++ |
| m28 | I28R + L142R | 30°C | +++ |
| m29 | C76Y + L142R | 30°C | ++++ |
| m30 | V31E + L142R | 30°C | ++++ |
| m31 | F246Y + L142R | 30°C | +++ |
| m33 | I28P + C76Y | 30°C | ++++ |
| m34 | I28P + C76Y + L142R | 30°C | +++++ |
| m35 | I28P + V31E + L142R | 30°C | ++++ |
| m36 | I28P + L142R + F246Y | 30°C | ++++ |

As is evident from Table 4 and Table 5, the wild-type enzyme XdPH expressed slight L-pipecolic acid hydroxylation activity when the recombinant *E*. *coli* was cultured at low temperature (a temperature around 15°C to 20°C), but did not express L-pipecolic acid hydroxylation activity at all when the recombinant *E*. *coli* was cultured at a temperature around 28°C to 30°C, at which *E. coli* grows well.

As is evident from Table 4, when the L-pipecolic acid hydroxylation activity after the low-temperature culture (15°C) was evaluated for the XdPH mutants to each of which a single mutation was introduced, improved L-pipecolic acid hydroxylation activity compared to that of the wild-type enzyme XdPH were found for the mutants having the amino acid sequence that, in the amino acid sequence of SEQ ID NO:2, the glutamic acid at position 15 is substituted by alanine (m9); the isoleucine at position 28 is substituted by proline (m10); the isoleucine at position 28 is substituted by arginine (m11); the valine at position 31 is substituted by glutamic acid (m12); the cysteine at position 76 is substituted by tyrosine (m13); the isoleucine at position 108 is substituted by arginine (m14); the leucine at position 142 is substituted by arginine (m15); or the phenylalanine at position 246 is substituted by tyrosine (m23). In particular, m15 had an especially improved L-pipecolic acid hydroxylation activity.

Further improvement of the L-pipecolic acid hydroxylation activity was achieved by combination of multiple mutations (m26 to m36) each of which showed improvement of the L-pipecolic acid hydroxylation activity by the introduction of the single mutation. In particular, the mutants having the mutation in which the leucine at position 142 is substituted by arginine (m26 to m31, and m34 to m36), the mutation in which the isoleucine at position 28 is substituted by proline (m27, and m33 to m36), and/or the mutation in which the cysteine at position 76 is substituted by tyrosine (m29, m33, and m34), compared to the amino acid sequence of SEQ ID NO:2, were found to have considerably improved L-pipecolic acid hydroxylation activity. Among these, the mutant (m34) in which the leucine at position 142 is substituted by arginine; the isoleucine at position 28 is substituted by proline; and the cysteine at position 76 is substituted by tyrosine; was found to have a remarkably improved L-pipecolic acid hydroxylation activity.

As is evident from Table 5, when the L-pipecolic acid hydroxylation activity after the normal culture (28°C or 30°C) was evaluated for the XdPH mutants to each of which a single mutation was introduced, m15, and m26 to m36 showed favorable expression of the L-pipecolic acid hydroxylation activity although the wild-type enzyme XdPH did not show expression of L-pipecolic acid hydroxylation activity. In particular, the mutants m26 to m36, to each of which multiple mutations were introduced, showed extremely high L-pipecolic acid hydroxylation activity.

Further improvement of the L-pipecolic acid hydroxylation activity was achieved by combination of multiple mutations (m26 to m36) each of which showed improvement of the L-pipecolic acid hydroxylation activity by the introduction of the single mutation. In particular, the mutants having the mutation in which the leucine at position 142 is substituted by arginine (m26 to m31, and m34 to m36), the mutation in which the isoleucine at position 28 is substituted by proline (m27, and m33 to m36), and/or the mutation in which the cysteine at position 76 is substituted by tyrosine (m29, m33, and m34), compared to the amino acid sequence of SEQ ID NO:2, were found to have considerably improved L-pipecolic acid hydroxylation activity. Among these, the mutant (m34) in which the leucine at position 142 is substituted by arginine; the isoleucine at position 28 is substituted by proline; and the cysteine at position 76 is substituted by tyrosine; was found to have a remarkably improved L-pipecolic acid hydroxylation activity.

Further, m29 and m30, and m33 to m36 were found to have higher L-pipecolic acid hydroxylation activity in the case where they were cultured at a temperature around 28°C to 30°C, at which *E. coli* grows well, than in the case where low-temperature culture was carried out.

The mutants XdPHm1 to XdPHm8, XdPHm 16 to XdPHm22, XdPHm24, and XdPHm25 hardly expressed, or did not express, the L-pipecolic acid hydroxylation activity either in the low-temperature culture or normal culture.

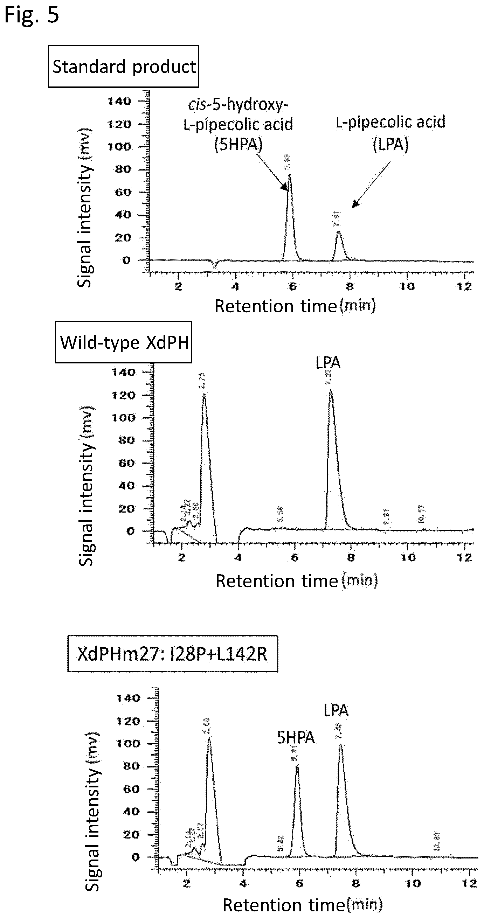

Fig. 5 shows the results (charts) of HPLC analysis for measuring the L-pipecolic acid hydroxylation activity of the wild-type enzyme XdPH and the mutant XdPHm27 that were subjected to normal culture. As can be seen from Fig. 5, *cis*-5-hydroxy-L-pipecolic acid was produced from the mutant XdPHm27.

### <Example 5: Evaluation of Activity of XdPH Mutants 2>

### (1) Providing of Enzyme Liquids

In order to obtain bacterial cells expressing the introduced gene, each recombinant *E. coli* obtained in Example 3 (the recombinant *E. coli* obtained by transformation using each of the wild-type plasmid pJ411XdPH, and the mutant expression plasmids pJ411XdPH_m34, and pJ411XdPH _m37 to pJ411XdPH_m59) was precultured at 30°C for about 5 hours using 1 mL of liquid LB medium supplemented with kanamycin and a *lac* promoter inducer. Thereafter, low-temperature culture (culture at 15°C or 20°C for about 24 hours) was carried out, and the bacterial cells were collected.

Each culture liquid after the culture was centrifuged to collect the bacterial cells, and the cells were suspended in 0.5 mL of 50 mmol/L MES (2-morpholinoethanesulfonic acid) buffer at pH 7. A container containing 0.5 mL of the obtained suspension was placed in ice water, and sonication was carried out, followed by carrying out centrifugation at a rotation speed of 12,000 rpm to separate the supernatant and the residue from each other. The obtained supernatant was used as an enzyme liquid for evaluation of the L-pipecolic acid hydroxylation activity.

### (2) Evaluation of Activity

In a plastic tube, 0.1 mL of a reaction liquid prepared by mixing components at the concentrations shown in Table 6 was placed. The tube was then shaken at 30°C for 30 minutes.

**Table 6**

| Component | Concentration |
|---|---|
| L-Pipecolic acid | 20 mmol/L |
| 2-Oxoglutaric acid | 40 mmol/L |
| L-Ascorbic acid | 25 mmol/L |
| Iron sulfate | 0.5 mmol/L |
| Each enzyme liquid provided in (1) above | About 2 mg/mL in terms of total protein concentration |

The reaction was stopped by transferring 20 µL of the reaction liquid after the shaking to another plastic tube containing 10 µL of hydrochloric acid having a concentration of 1 mmol/L. Thereafter, 10 µL of aqueous sodium hydroxide solution having a concentration of 1 mmol/L was added thereto to neutralize the reaction liquid. Further, 40 µL of aqueous copper sulfate solution having a concentration of 2 mmol/L was added thereto, and then the precipitate was removed by centrifugation. To 20 µL of the obtained supernatant, 20 µL of borate buffer (pH 9.0) having a concentration of 1 mol/L and 50 µL of a solution of FDAA (1-fluoro-2,4-dinitrophenyl-5-L-alanine amide) having a concentration of 20 mmol/L in acetonitrile were added, and the reaction was allowed to proceed at 40°C for 60 minutes.

The reaction was stopped by addition of 10 µL of hydrochloric acid having a concentration of 1 mol/L, and then 100 µL of methanol was added, followed by performing centrifugation to remove the precipitate. Thereafter, 150 µL of the obtained supernatant was analyzed by UPLC (registered trademark) (Ultra Performance LC) under the following conditions, to measure the concentration of the produced cis-5-hydroxy-L-pipecolic acid.

### <UPLC (Registered Trademark) Analysis Conditions>

Apparatus used: ACQUITY UPLC (registered trademark) System (manufactured by Waters)
Column: ACQUITY UPLC BEH C18 (1.7 µm) 2.1 × 100 mm (manufactured by Waters)
Detection wavelength: 340 nm
Injection volume: 5 µL
Eluent A: 0.1% formic acid
Eluent B: acetonitrile (0.1% formic acid)
Flow rate: 0.2 mL/min
Gradient conditions:

| Time (minute) | | Eluent B (%) | |
|---|---|---|---|
| | 0 | 20 | |
| | 12 | 55 | |
| | 12.5 | 100 | |
| | 14.5 | 100 | |
| | 14.6 | 20 | |
| | 17.8 | 20 | |

The evaluation results on the L-pipecolic acid hydroxylation activity of the wild-type enzyme XdPH and the XdPH mutants are shown in Table 7.

In Table 7, the L-pipecolic acid hydroxylation activity was evaluated as a relative activity to that of the wild-type enzyme XdPH, wherein the production (peak area) of *cis*-5-hydroxy-L-pipecolic acid obtained by the reaction using the wild-type enzyme XdPH was taken as 1. In Table 7, the symbols indicating the L-pipecolic acid hydroxylation activity have the following meanings.

| Symbol | Relative activity |
|---|---|
| 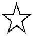 | 0 to 2 |
| 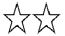 | 2 to 5 |
| 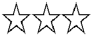 | 5 to 10 |
| 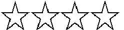 | >10 |

**Table 7**

| Mutant No. | Mutation site | | Culture Temp. | L-Pipecolic acid hydroxylation activity |
|---|---|---|---|---|
| Wild-type XdPH | | (No mutation) | 15°C | (Standard) |
| | | | 20°C | ☆ |
| m34 | I28P + C76Y + L142R | | 15°C | ☆☆☆☆ |
| | | | 20°C | ☆☆☆☆ |
| m37 | A32E | | 15°C | ☆ |
| m38 | I44H | | 15°C | ☆ |
| m39 | S98L | | 15°C | ☆ |
| m40 | Y105I | | 15°C | ☆ |
| m41 | V123P | | 15°C | ☆ |
| m42 | V163P | | 15°C | ☆ |
| m43 | Y166L | | 15°C | ☆ |
| m44 | K177A | | 15°C | ☆ |
| m45 | V190G | | 15°C | ☆ |
| m46 | I197R | | 15°C | ☆ |
| m47 | H199V | | 15°C | ☆ |
| m48 | F222E | | 15°C | ☆ |
| m49 | A229R | | 15°C | ☆ |
| m50 | A239D | | 15°C | ☆☆ |
| m51 | I232E | | 15°C | ☆ |
| m52 | D75P | | 20°C | ☆ |
| m53 | G151A | | 20°C | ☆ |
| m54 | A154P | | 20°C | ☆ |
| m55 | K165R | | 20°C | ☆ |
| m56 | Q202P | | 20°C | ☆☆ |
| m57 | C252I | | 20°C | ☆ |
| m58 | P257G | | 20°C | ☆ |
| m59 | Y258D | | 20°C | ☆ |

As is evident from Table 7, when the L-pipecolic acid hydroxylation activity after the low-temperature culture (15°C or 20°C) was evaluated for the XdPH mutants to each of which a single mutation was introduced, improved L-pipecolic acid hydroxylation activity compared to that of the wild-type enzyme XdPH were found for the mutants having the amino acid sequence that, in the amino acid sequence of SEQ ID NO:2, the alanine at position 239 is substituted by aspartic acid (m50), or the glutamine at position 202 is substituted by proline (m56).

Further, m34, in which multiple (three) mutations were introduced, showed a remarkably improved L-pipecolic acid hydroxylation activity compared to that of the wild-type enzyme XdPH.

Among the XdPH mutants to each of which a single mutation was introduced, m37 to m49, m51 to m55, and m57 to m59 did not show an improved L-pipecolic acid hydroxylation activity compared to that of the wild-type enzyme XdPH.

### <Example 6: Evaluation of Solubility of XdPH Mutants 1>

### (1) Providing of Solutions for Evaluation of Solubility

In order to obtain bacterial cells expressing the introduced gene, each recombinant *E. coli* obtained in Example 3 (the recombinant *E. coli* obtained by transformation using each of the wild-type plasmid pJ411XdPH, and the mutant expression plasmids pJ411XdPH_m1 to pJ411XdPH_m23, pJ411XdPH_m26 to pJ411XdPH_m30, and pJ411XdPH_m34) was precultured at 30°C for about 5 hours using 1 mL of liquid LB medium supplemented with kanamycin and a *lac* promoter inducer. Thereafter, normal culture (culture at 28°C for about 20 hours) or low-temperature culture (culture at 15°C for about 24 hours) was carried out, and then centrifugation at a rotation speed of 12,000 rpm was carried out to collect the bacterial cells.

The whole amount of each obtained recombinant *E. coli* was suspended in 50 mmol/L MES buffer at pH 7 such that the turbidity (OD630) was about 10. A container containing 0.5 mL of the obtained suspension was placed in ice water, and sonication was carried out, followed by carrying out centrifugation at a rotation speed of 12,000 rpm to separate the supernatant and the residue from each other. The obtained supernatant was provided as a soluble fraction, and the residue was provided as an insoluble fraction.

### (2) Evaluation of Solubility

Each obtained soluble fraction was subjected to quantification of the protein concentration according to a conventional method. Thereafter, the fraction was transferred to a plastic tube such that the total protein mass was 10 µg, and suspended in an SDS (sodium dodecyl sulfate)-containing solubilization buffer, followed by heating at 90°C for about 10 minutes to provide a solution for evaluation of the solubility.

Each obtained insoluble fraction was suspended in 0.5 mL of the SDS-containing solubilization buffer, and then solubilized by heating at 90°C for 10 minutes to provide a solution, which was used as a solution for evaluation of the solubility.

Each obtained solution was subjected to SDS-polyacrylamide electrophoresis according to a conventional method, to investigate the amount of expressed XdPH protein.

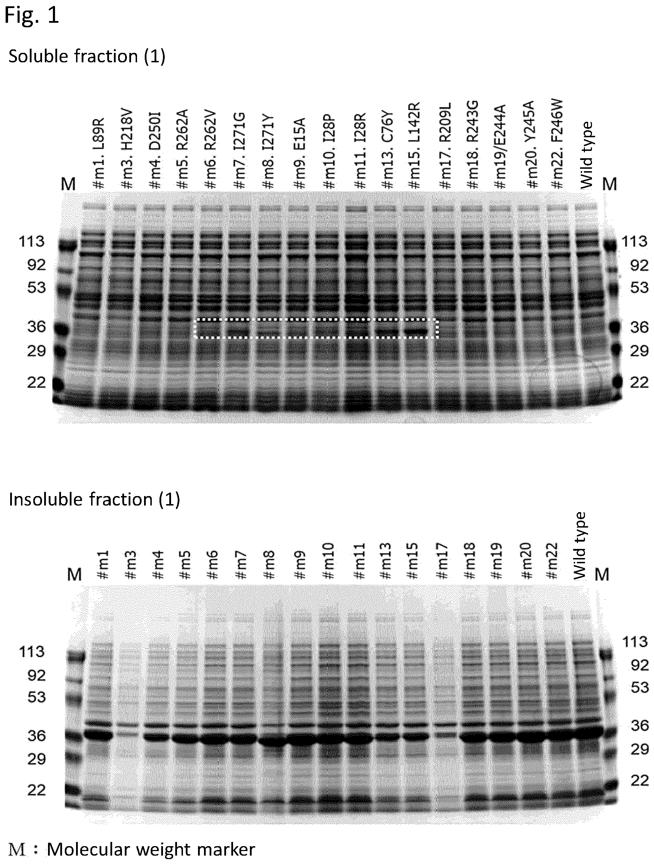

Table 8 and Fig. 1 show the results of evaluation of the solubility after the low-temperature culture of the XdPH mutants to each of which a single mutation was introduced.

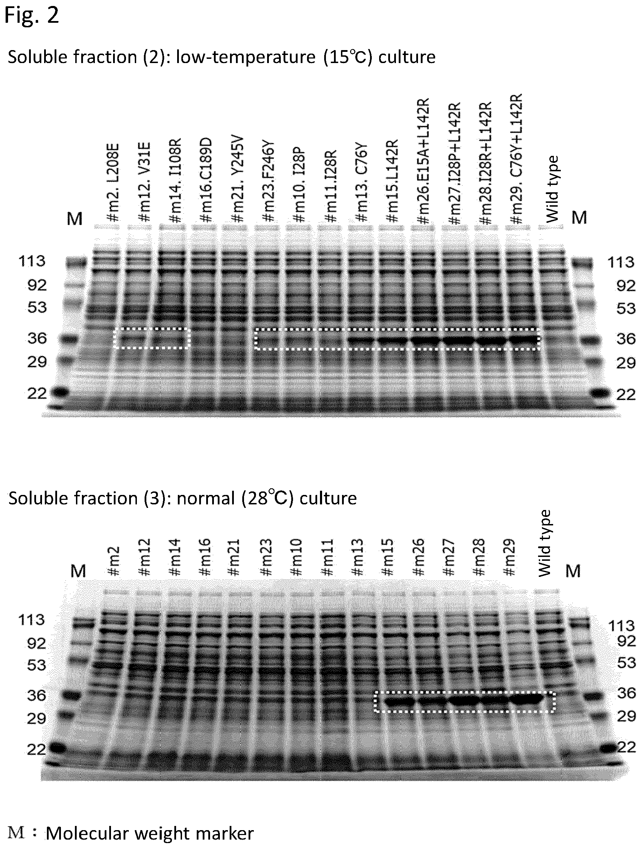

Table 9 and Fig. 2 show the results of evaluation of the solubility after the low-temperature culture or normal culture of the XdPH mutants to each of which a single mutation or multiple mutations was/were introduced.

In the evaluation of the solubility, the result of the electrophoresis was visually observed, and rating was performed using as an index the solubility level which was determined using the solubility expression level of the wild-type enzyme XdPH as a standard. In Table 8 and Table 9, the symbols indicating the solubility level have the following meanings.

| Symbol | Solubility level |
|---|---|
| - | Not more than the level of the wild type |
| * | Slightly improved solubility compared to the wild type |
| ** | Rather improved solubility compared to the wild type |
| *** | Remarkably improved solubility compared to the wild type |

**Table 8**

| Mutant No. | Mutation site | | Solubility level |
|---|---|---|---|
| | | | Culture Temp.: 15°C |
| Wild-type XdPH | | (No mutation) | (Standard) |
| m1 | L89R | | - |
| m3 | H218V | | - |
| m4 | D250I | | - |
| m5 | R262A | | - |
| m6 | R262V | | ∗ |
| m7 | I271G | | ∗∗ |
| m8 | 1271Y | | ∗ |
| m9 | E15A | | ∗ |
| m10 | I28P | | ∗∗ |
| m11 | 128R | | ∗∗ |
| m13 | C76Y | | ∗∗ |
| m15 | L142R | | ∗∗ |
| m17 | R209L | | - |
| m18 | R243G | | - |
| m19 | E244A | | - |
| m20 | Y245A | | - |
| m22 | F246W | | - |

**Table 9**

| Mutant No. | Mutation site | | Solubility level | |
|---|---|---|---|---|
| | | | Culture Temp. | |
| | | | 15°C | 28°C |
| Wild-type XdPH | | (No mutation) | (Standard) | (Standard) |
| m2 | L208E | | - | - |
| m10 | 128P | | ∗ ∗ | ∗ ∗ |
| m11 | I28R | | ∗ ∗ | ∗ ∗ |
| m12 | V31E | | ∗ ∗ | ∗ ∗ |
| m13 | C76Y | | ∗ ∗ | ∗ ∗ |
| m14 | I108R | | ∗ | ∗ |
| m15 | L142R | | ∗ ∗ | ∗ ∗ ∗ |
| m16 | C189D | | - | - |
| m21 | Y245V | | - | - |
| m23 | F246Y | | ∗ ∗ | ∗ ∗ |
| m26 | E15A + L142R | | ∗ ∗ ∗ | ∗ ∗ ∗ |
| m27 | I28P + L142R | | ∗ ∗ ∗ | ∗ ∗ ∗ |
| m28 | I28R + L142R | | ∗ ∗ ∗ | ∗ ∗ ∗ |
| m29 | C76Y + L142R | | ∗ ∗ ∗ | ∗ ∗ ∗ |
| m30 | V31E + L142R | | ∗ ∗ ∗ | - |
| m34 | I28P + C76Y + L142R | | ∗ ∗ ∗ | - |

The wild-type enzyme XdPH slightly expresses solubility as an active type in *E. coli* when the recombinant *E*. *coli* is subjected to low-temperature culture. However, expression of the solubility as an active type in *E. coli* is difficult for the protein when the recombinant *E. coli* is cultured at a temperature around 28°C to 30°C, at which *E. coli* grows well.

When the solubility after the low-temperature culture (15°C) was evaluated for the XdPH mutants shown in Table 8, to each of which a single mutation was introduced, slightly improved solubility compared to that of the wild-type enzyme XdPH were found for the mutants having the amino acid sequence that, in the amino acid sequence of SEQ ID NO:2, the arginine at position 262 is substituted by valine (m6); the isoleucine at position 271 is substituted by tyrosine (m8); the glutamic acid at position 15 is substituted by alanine (m9); or the isoleucine at position 108 is substituted by arginine (m14).

Rather improved solubility compared to that of the wild-type enzyme XdPH were found for the mutants having the amino acid sequence that, in the amino acid sequence of SEQ ID NO:2, the isoleucine at position 271 is substituted by glycine (m7); the isoleucine at position 28 is substituted by proline (m10); the isoleucine at position 28 is substituted by arginine (m11); the valine at position 31 is substituted by glutamic acid (m12); the cysteine at position 76 is substituted by tyrosine (m13); the leucine at position 142 is substituted by arginine (m15); or the phenylalanine at position 246 is substituted by tyrosine (m23).

Evaluation of the solubility after low-temperature culture (15°C) or normal culture (28°C) was carried out for the XdPH mutants shown in Table 9, to each of which a single mutation or multiple mutations was/were introduced.

Among the XdPH mutants to each of which a single mutation was introduced, the mutant having the amino acid sequence that, in the amino acid sequence of SEQ ID NO:2, the isoleucine at position 108 is substituted by arginine (m14) showed slightly improved solubility compared to that of the wild-type enzyme XdPH both after the low-temperature culture (15°C) and the normal culture (28°C).

Among the XdPH mutants to each of which a single mutation was introduced, the mutants having the amino acid sequence that, in the amino acid sequence of SEQ ID NO:2, the isoleucine at position 28 is substituted by proline (m10); the isoleucine at position 28 is substituted by arginine (m11); the valine at position 31 is substituted by glutamic acid (m12); the cysteine at position 76 is substituted by tyrosine (m13); or the phenylalanine at position 246 is substituted by tyrosine (m23); showed rather improved solubility compared to that of the wild-type enzyme XdPH both after the low-temperature culture (15°C) and the normal culture (28°C).

Among the XdPH mutants to each of which a single mutation was introduced, the mutant having the amino acid sequence that, in the amino acid sequence of SEQ ID NO:2, the leucine at position 142 is substituted by arginine (m15) showed a rather improved solubility compared to that of the wild-type enzyme XdPH after the low-temperature culture (15°C), and a remarkably improved solubility compared to that of the wild-type enzyme XdPH after the normal culture (28°C).

m26 to m30, and m34, to each of which multiple mutations were introduced, showed remarkably improved solubility compared to that of the wild-type enzyme XdPH after the low-temperature culture (15°C). m26 to m29, to each of which multiple mutations were introduced, showed remarkably improved solubility compared to that of the wild-type enzyme XdPH also after the normal culture (28°C). It was thus confirmed that the efficiency of solubility expression can be increased by introduction of multiple mutations.

Among the XdPH mutants shown in Table 8 and Table 9, m1 to m5, and m16 to m22 had lower solubility than that of the wild-type enzyme XdPH.

### <Example 7: Evaluation of Solubility of XdPH Mutants 2>

### (1) Providing of Enzyme Liquids

In order to obtain bacterial cells expressing the introduced gene, each recombinant *E. coli* obtained in Example 3 (the recombinant *E. coli* obtained by transformation using each of the wild-type plasmid pJ411XdPH, and the mutant expression plasmids pJ411XdPH_m34, and pJ411XdPH _m37 to pJ411XdPH_m59) was precultured at 30°C for about 5 hours using 1 mL of liquid LB medium supplemented with kanamycin and a *lac* promoter inducer. Thereafter, low-temperature culture (culture at 15°C or 20°C for about 24 hours) was carried out, and then centrifugation at a rotation speed of 12,000 rpm was carried out to collect the bacterial cells.

The whole amount of each obtained recombinant *E. coli* was suspended in 0.5 mL of 50 mmol/L MES buffer at pH 7. A container containing the obtained suspension was placed in ice water, and sonication was carried out, followed by carrying out centrifugation at a rotation speed of 12,000 rpm to separate the supernatant and the residue from each other. The obtained supernatant was provided as a soluble fraction, and the residue was provided as an insoluble fraction.

### (2) Evaluation of Solubility

To a plastic tube, 20 µL of each obtained soluble fraction was transferred. The fraction was suspended in the SDS-containing solubilization buffer, and then heated at 100°C for about 10 minutes to provide a solution for evaluation of the solubility.

Each obtained insoluble fraction was suspended in 0.5 mL of the SDS-containing solubilization buffer, and then solubilized by heating at 100°C for 10 minutes to provide a solution, which was used as a solution for evaluation of the solubility.

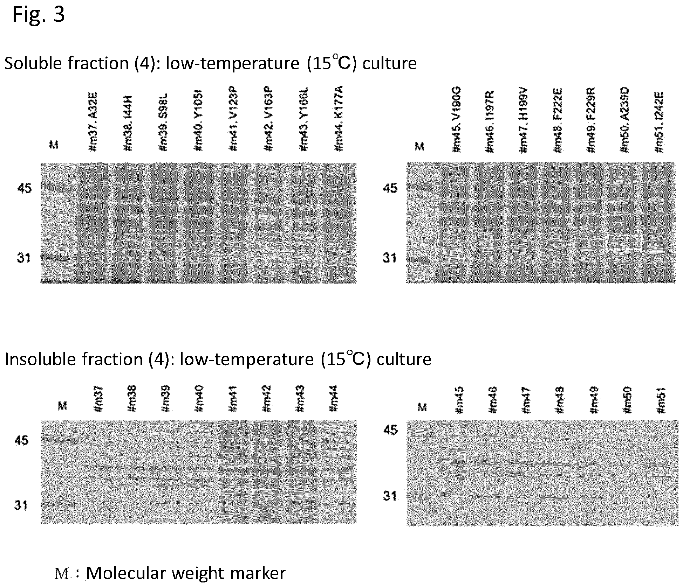

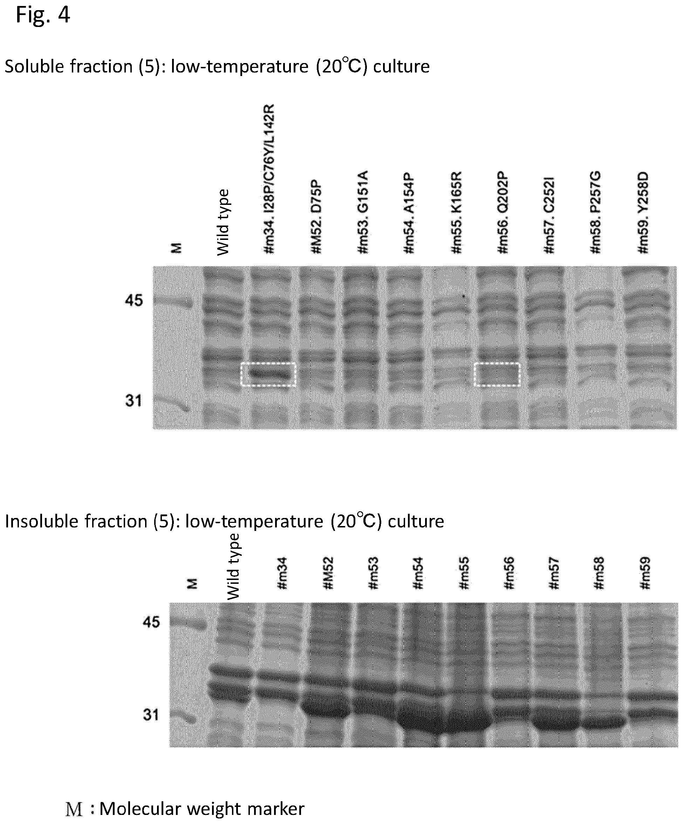

Each obtained solution was subjected to SDS-polyacrylamide electrophoresis according to a conventional method, to investigate the amount of expressed XdPH protein. The results of the evaluation of the solubility are shown in Table 10, Fig. 3, and Fig. 4.

In the evaluation of the solubility, the result of the electrophoresis was visually observed, and rating was performed using as an index the solubility level which was determined using the solubility expression level of the wild-type enzyme XdPH as a standard. In Table 10, the symbols indicating the solubility level have the following meanings.

| Symbol | Solubility level |
|---|---|
| - | Not more than the level of the wild type |
| ** | Rather improved solubility compared to the wild type |
| *** | Remarkably improved solubility compared to the wild type |

**Table 10**

| No. | Mutation site | | Culture Temp. | | Solubility level |
|---|---|---|---|---|---|
| Wild-type XdPH | | (No mutation) | 20°C | | (Standard) |
| m34 | I28P + C76Y + L142R | | 20°C | ∗∗∗ | |
| m37 | A32E | | 15°C | - | |
| m38 | I44H | | 15°C | - | |
| m39 | S98L | | 15°C | - | |
| m40 | Y105I | | 15°C | - | |
| m41 | V123P | | 15°C | - | |
| m42 | V163P | | 15°C | - | |
| m43 | Y166L | | 15°C | - | |
| m44 | K177A | | 15°C | - | |
| m45 | V190G | | 15°C | - | |
| m46 | I197R | | 15°C | - | |
| m47 | H199V | | 15°C | - | |
| m48 | F222E | | 15°C | - | |
| m49 | A229R | | 15°C | - | |
| m50 | A239D | | 15°C | ∗∗ | |
| m51 | I232E | | 15°C | - | |
| m52 | D75P | | 20°C | - | |
| m53 | G151A | | 20°C | - | |
| m54 | A154P | | 20°C | - | |
| m55 | K165R | | 20°C | - | |
| m56 | Q202P | | 20°C | ∗∗ | |
| m57 | C252I | | 20°C | - | |
| m58 | P257G | | 20°C | - | |
| m59 | Y258D | | 20°C | - | |

Among the XdPH mutants to each of which a single mutation was introduced, rather improved solubility compared to that of the wild-type enzyme XdPH were found for the mutants having the amino acid sequence that, in the amino acid sequence of SEQ ID NO:2, the alanine at position 239 is substituted by aspartic acid (m50), or the glutamine at position 202 is substituted by proline (m56).

Further, m34, to which multiple mutations were introduced, showed a remarkably improved solubility compared to that of the wild-type enzyme XdPH, indicating that the efficiency of solubility expression can be increased by introduction of multiple mutations.

Among the XdPH mutants shown in Table 10, m37 to m49, m51 to m55, and m57 to m59 had lower solubility than that of the wild-type enzyme XdPH.

### <Example 8: Introduction of Mutations to XdPH Gene 3>

Using the plasmid pJ411XdPH obtained in Example 1 as a template, the plasmid pET28aXdPH, which has an insertion in the plasmid pET-28a(+) (manufactured by Merck Millipore), was prepared according to a conventional method. Further, using the obtained pET28aXdPH as a template, the mutant expression plasmids shown in Table 11, each of which has a site-specific modification of the amino acid at position 142, (Nos. m 15, and m60 to m77) were prepared according to a conventional method.

**Table 11: Prepared mutant expression plasmids**

| Mutant No. | Gene mutation site | Template plasmid | Mutant expression plasmid |
|---|---|---|---|
| m15 | L142R | pET28aXdPH | pET28aXdPH_m15 |
| m60 | L142K | pET28aXdPH | pET28aXdPH_m60 |
| m61 | L142N | pET28aXdPH | pET28aXdPH_m61 |
| m62 | L142D | pET28aXdPH | pET28aXdPH_m62 |
| m63 | L142Q | pET28aXdPH | pET28aXdPH_m63 |
| m64 | L142E | pET28aXdPH | pET28aXdPH_m64 |
| m65 | L142H | pET28aXdPH | pET28aXdPH_m65 |
| m66 | L142P | pET28aXdPH | pET28aXdPH_m66 |
| m67 | L142Y | pET28aXdPH | pET28aXdPH_m67 |
| m68 | L142S | pET28aXdPH | pET28aXdPH_m68 |
| m69 | L142W | pET28aXdPH | pET28aXdPH_m69 |
| m70 | L142T | pET28aXdPH | pET28aXdPH_m70 |
| m71 | L142G | pET28aXdPH | pET28aXdPH_m71 |
| m72 | L142A | pET28aXdPH | pET28aXdPH_m72 |
| m73 | L142M | pET28aXdPH | pET28aXdPH_m73 |
| m74 | L142C | pET28aXdPH | pET28aXdPH_m74 |
| m75 | L142F | pET28aXdPH | pET28aXdPH_m75 |
| m76 | L142V | pET28aXdPH | pET28aXdPH_m76 |
| m77 | L142I | pET28aXdPH | pET28aXdPH_m77 |

### <Example 9: Obtaining of Mutant XdPH Gene-Expressing Bacterial Cells 2>

Using each of the wild-type plasmid pET28aXdPH and the mutant expression plasmids obtained in Example 8, *E. coli* (*Escherichia coli*) BL21(DE3) (manufactured by Invitrogen) was transformed according to a conventional method, to obtain each recombinant *E. coli.*

### <Example 10: Evaluation of Activity of XdPH Mutants 3>

### (1) Providing of Enzyme Liquids

In order to obtain bacterial cells expressing the introduced gene, each recombinant *E. coli* obtained in Example 9 (the recombinant *E. coli* obtained by transformation using each of the wild-type plasmid pET28aXdPH, and the mutant expression plasmids pET28aXdPH_m15, and pET28aXdPH_m60 to pET28aXdPH_m77) was cultured using LB autoinduction medium (manufactured by Novagen) at 30°C until OD₆₀₀ reached 0.6 to 0.8, followed by performing culture at 15°C for about 24 hours, and then collecting the bacterial cells.

Subsequently, 2 mL of each recombinant *E. coli* was centrifuged to collect the bacterial cells, and the collected bacterial cells were suspended in 100 mmol/L MES buffer at pH 6.5. A container containing 0.5 mL of the obtained suspension was placed in ice water, and sonication was carried out, followed by centrifugation at a rotation speed of 20,000 rpm. The obtained supernatant was used as an enzyme liquid for evaluation of the L-pipecolic acid hydroxylation activity.

### (2) Evaluation of Activity

In a plastic tube, 0.05 mL of a reaction liquid prepared by mixing components at the concentrations shown in Table 12 was placed. The tube was then left to stand at 20°C for 10 minutes to allow the reaction to proceed.

**Table 12**

| Component | Concentration |
|---|---|
| L-Pipecolic acid | 20 mmol/L |
| 2-Oxoglutaric acid | 40 mmol/L |
| L-Ascorbic acid | 1 mmol/L |
| Citric acid | 2 mmol/L |
| Iron sulfate | 0.5 mmol/L |
| MES (pH6.5) | 150 mmol/L |
| Each enzyme liquid provided in (1) above | About 1 mg/mL in terms of total protein concentration |

In another plastic tube containing 25 µL of a solution of FDLA (1-fluoro-2,4-dinitrophenyl-L-leucinamide) having a concentration of 31.8 mmol/L in acetone, 12.5 µL of the obtained reaction liquid was placed to stop the reaction, and then 5 µL of sodium carbonate having a concentration of 1 mol/L was added thereto, followed by allowing the reaction to proceed at 37°C for 60 minutes. After stopping the reaction by addition of 5 µL of hydrochloric acid having a concentration of 2 mol/L, the reaction liquid was diluted by addition of 427.5 µL of an eluent (3.83 mol/L aqueous acetonitrile solution containing 26.5 mmol/L formic acid). The obtained dilution was filtered to remove the precipitate. The filtrate was analyzed by UPLC (registered trademark) under the same conditions as in Example 5, to measure the concentration of the produced cis-5-hydroxy-L-pipecolic acid.

The evaluation results on the L-pipecolic acid hydroxylation activity of the wild-type XdPH and the XdPH mutants (m15, and m60 to m77) are shown in Table 13.

In Table 13, the L-pipecolic acid hydroxylation activity was evaluated as the relative amount of production (relative activity) to the amount of cis-5-hydroxy-L-pipecolic acid produced (U/g-total protein) per total protein mass (unit: g) obtained by the reaction using the wild-type enzyme XdPH. The unit (U) herein represents the capacity to produce 1 µmol of cis-5-hydroxy-L-pipecolic acid per minute. In Table 13, the symbols indicating the L-pipecolic acid hydroxylation activity have the following meanings.

| Symbol | Relative activity |
|---|---|
| 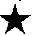 | 0 to 2 |
| 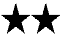 | 2 to 5 |
| 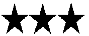 | 5 to 10 |
| 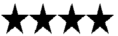 | >10 |

**Table 13**

| Mutant No. | Mutation site | L-Pipecolic acid hydroxylation activity |
|---|---|---|
| | | Culture Temp. : 15°C |
| Wild-type XdPH | (No mutation) | (Standard) |
| m15 | L142R | 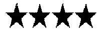 |
| m60 | L142K | 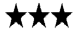 |
| m61 | L142N | 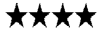 |
| m62 | L142D | 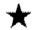 |
| m63 | L142Q | 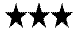 |
| m64 | L142E | 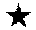 |
| m65 | L142H | 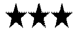 |
| m66 | L142P | 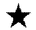 |
| m67 | L142Y | 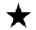 |
| m68 | L142S | 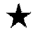 |
| m69 | L142W | 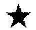 |
| m70 | L142T | 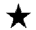 |
| m71 | L142G | 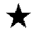 |
| m72 | L142A | 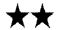 |
| m73 | L142M | 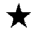 |
| m74 | L142C | 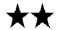 |
| m75 | L142F | 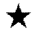 |
| m76 | L142V | 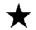 |
| m77 | L142I | 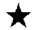 |

Among the XdPH mutants to each of which a single mutation was introduced, improved L-pipecolic acid hydroxylation activity compared to that of the wild-type enzyme XdPH were found for m15, m60, m61, m63, m65, m72, and m74, in which the leucine at position 142 is substituted by arginine, lysine, asparagine, glutamine, histidine, alanine, or cysteine, respectively. Among these, especially improved L-pipecolic acid hydroxylation activity was found for m15, m60, m61, m63, and m65, in which the leucine at position 142 is substituted by arginine, lysine, asparagine, glutamine, or histidine, respectively.

The L-pipecolic acid hydroxylation activity tends to be improved by substituting leucine, which is an amino acid having high hydrophobicity, by arginine, lysine, asparagine, glutamine, histidine, alanine, or cysteine, preferably by arginine, lysine, asparagine, glutamine, or histidine, which are amino acids having lower hydrophobicity (higher hydrophilicity) than leucine.

Substitution to negatively charged amino acids (m64: glutamic acid; m62: aspartic acid) did not result in improvement of the L-pipecolic acid hydroxylation activity even though the amino acids have low hydrophobicity.

As a result of modeling of the crystal structure of the wild-type enzyme XdPH by SWISS-MODEL (https://swissmodel.expasy.org/), the leucine at position 142 was found to be positioned on the enzyme surface. It is thus thought that the substitutions from an amino acid having high hydrophobicity to an amino acid having low hydrophobicity led to increased solubility in water, resulting in an increased expression level as a soluble protein, and hence improvement of the L-pipecolic acid hydroxylation activity.

### <Description of Sequence Listing>

SEQ ID NO: 1 XdPH gene sequence that is codon-optimized for *E. coli*
SEQ ID NO:2 XdPH amino acid sequence

## Claims

1. A mutant L-pipecolic acid hydroxylase having an amino acid sequence that at least one amino acid mutation selected from the group consisting of the following (a) to (o) is introduced in the amino acid sequence of SEQ ID NO:2:
(a) amino acid mutation in which the glutamic acid at position 15 is substituted by alanine;
(b) amino acid mutation in which the isoleucine at position 28 is substituted by proline;
(c) amino acid mutation in which the isoleucine at position 28 is substituted by arginine;
(d) amino acid mutation in which the valine at position 31 is substituted by glutamic acid;
(e) amino acid mutation in which the cysteine at position 76 is substituted by tyrosine;
(f) amino acid mutation in which the isoleucine at position 108 is substituted by arginine;
(g) amino acid mutation in which the leucine at position 142 is substituted by arginine;
(h) amino acid mutation in which the leucine at position 142 is substituted by lysine;
(i) amino acid mutation in which the leucine at position 142 is substituted by asparagine;
(j) amino acid mutation in which the leucine at position 142 is substituted by glutamine;
(k) amino acid mutation in which the leucine at position 142 is substituted by histidine;
(l) amino acid mutation in which the glutamine at position 202 is substituted by proline;
(m) amino acid mutation in which the alanine at position 239 is substituted by aspartic acid;
(n) amino acid mutation in which the phenylalanine at position 246 is substituted by tyrosine; and
(o) amino acid mutation in which the isoleucine at position 271 is substituted by glycine.

2. The mutant L-pipecolic acid hydroxylase according to claim 1, having an amino acid sequence that at least two amino acid mutations selected from the group consisting of the (a) to (o) are introduced in the amino acid sequence of SEQ ID NO:2.

3. The mutant L-pipecolic acid hydroxylase according to claim 1 or 2, having an amino acid sequence that at least three amino acid mutations selected from the group consisting of the (a) to (o) are introduced in the amino acid sequence of SEQ ID NO:2.

4. A method of producing cis-5-hydroxy-L-pipecolic acid, the method comprising bringing the mutant L-pipecolic acid hydroxylase according to any one of claims 1 to 3, a microorganism or cell having a capacity to produce the enzyme, a processed product of the microorganism or cell, and/or a culture liquid containing the enzyme obtained by culturing the microorganism or cell, into contact with L-pipecolic acid, to produce cis-5-hydroxy-L-pipecolic acid.

5. The method of producing cis-5-hydroxy-L-pipecolic acid according to claim 4, comprising bringing the mutant L-pipecolic acid hydroxylase, a microorganism or cell having a capacity to produce the enzyme, a processed product of the microorganism or cell, and/or a culture liquid containing the enzyme obtained by culturing the microorganism or cell, into contact with L-pipecolic acid in the presence of 2-oxoglutaric acid and ferrous ions.
